# EUROPÄISCHE PATENTSCHRIFT

(11) **EP 2 491 955 B1**
(45) Veröffentlichungstag und Bekanntmachung des Hinweises auf die Patenterteilung: **11.01.2017**
(21) Anmeldenummer: 12156546.9
(22) Anmeldetag: 22.02.2012
(51) Int. Cl.: A61L 2/08, B65B 55/02, B67C 7/00

(54) **Verfahren und Vorrichtung zur Sterilisation von Behältnissen**
Method and device for sterilising containers
Procédé et dispositif destinés à la stérilisation de récipients

(30) Priorität: 24.02.2011 DE 102011012342
(43) Veröffentlichungstag der Anmeldung: 29.08.2012
(73) Patentinhaber: Krones AG, 93073 Neutraubling (DE)
(72) Erfinder: Meinzinger, Rupert, 94356 Kirchroth (DE); Justl, Johann, 93049 Regensburg (DE)
(74) Vertreter: Bittner, Bernhard

(56) Entgegenhaltungen:
- EP-A1- 2 161 202
- EP-A1- 2 371 397
- WO-A1-2011/067393
- WO-A1-2011/080245
- WO-A2-2009/095182
- DE-A1-102009 008 633

## Beschreibung

Die vorliegende Erfindung bezieht sich auf ein Verfahren zum Sterilisieren von Behältnissen. Im Bereich der getränkeherstellenden Industrie ist es bekannt, dass Behältnisse, beispielsweise Kunststoffbehältnisse, vor deren Befüllung sterilisiert werden. Diese Sterilisierung kann dabei auf unterschiedlichste Art und Weise erfolgen. So ist es beispielsweise möglich, dass die Behältnisse mit einem Sterilisationsmittel, wie beispielsweise H₂O₂ (Wasserstoffperoxid) oder Peressigsäure sterilisiert werden. Der Umgang mit diesen Medien ist jedoch nicht unproblematisch, da diese auch für Menschen schädliche Wirkungen haben. Weiterhin ist es bei Verwendung dieser Medien erforderlich, im Nachgang das Behältnis zu spülen.

In jüngerer Zeit sind auch Verfahren bekannt geworden, bei denen Behältnisse mittels Ladungsträgerstrahlung und insbesondere Elektronenstrahlung sterilisiert werden. So beschreibt die DE 10 2008 007 428 A1 ein Verfahren und eine Vorrichtung zum Sterilisieren von Packmitteln. Dabei wird ein Behandlungskopf für die Behandlung einer Packmittelinnenfläche mit Elektronenstrahlung durch eine Packmittelöffnung in dem jeweiligen Packmittel platziert.

Die EP 1 982 921 A1 der Anmelderin beschreibt eine Vorrichtung zum Sterilisieren von Behältnissen. Diese Vorrichtung weist einen Behandlungskopf mit einem Austrittsfenster, durch welches Ladungsträger austreten können, auf. Der Offenbarungsgehalt der EP 1 982 921 A1 wird hiermit durch Bezugnahme vollständig zum Gegenstand der vorliegenden Offenbarung gemacht. Aus der DE 10 2008 025 868 A1 ist ebenfalls eine Vorrichtung zum Sterilisieren von Behältnissen mittels Ladungsträgern bekannt, welche insbesondere auch eine Kühleinrichtung für das Austrittsfenster für die Elektronen beschreibt. Auch der Offenbarungsgehalt der DE 10 2008 025 868 A1 wird hiermit durch Bezugnahme zum Gegenstand der vorliegenden Anmeldung gemacht.

Aus der EP 1 612 02 A2 ist eine Elektronenstrahlsterilisation für Behältnisse bekannt. Dabei wird eine Bewegungsgeschwindigkeit eines Strahlfingers im Inneren eines Behältnisses in Abhängigkeit von der geometrischen Gestalt des Behälters variiert.

Die EP 2 371 397 A1 beschreibt ebenfalls eine Vorrichtung zum Sterilisieren von Behältnissen. Dabei ist eine Innensterilisation der Behältnisse durch einen Strahlfinger sowie auch eine Außensterilisation der Behältnisse vorgesehen.

Die WO 2009/095182 A2 beschreibt ein Verfahren und eine Vorrichtung zum Sterilisieren von Packmitteln. Auch hier wird eine Innen- und eine Außensterilisation der Behältnisse vorgenommen.

Aus der WO 2011/080245 A1 ist ein Schutzsystem für eine Elektronenstrahlbehandlung bekannt. Dabei wird eine Abschirmung der Elektronenstrahlen bzw. der sich daraus ergebenen Röntgenstrahlen vorgenommen.

Die WO 2011/067393 A1 beschreibt eine Vorrichtung zum Schützen einer Behälterbehandlungsanlage. Dabei ist ein Schutzsystem vorgesehen, um die von den Emittern abgegebene Strahlung abzuschirmen.

Die DE 10 2009 008 633 A1 beschreibt eine Vorrichtung zum Sterilisieren von Behältnissen. Dabei ist ebenfalls ein Strahlfinger vorgesehen, der in das Innere der Behältnisse einführbar ist.

Teilweise ist jedoch die Sterilisation der Innenwandungen der Kunststoffbehältnisse nicht ausreichend für eine Gesamtsterilisation.

Der vorliegenden Erfindung liegt daher die Aufgabe zugrunde, eine Sterilisation der Kunststoffbehältnisse dahingehend zu ermöglichen, dass diese anschließend mit einer Flüssigkeit und insbesondere einem Getränk befüllt werden können, ohne dass ein weiterer oder vorhergehender Sterilisationsvorgang erfolgen muss.

Diese Aufgaben werden erfindungsgemäß durch Anspruch 1 gelöst.

Vorteilhafte Ausführungsformen und Weiterbildungen sind Gegenstand der Unteransprüche.

Erfindungsgemäß wird auch ein außenliegender Mündungsbereich des Behältnisses sterilisiert.

Unter dem außenliegenden Bereich wird dabei insbesondere ein Mündungsbereich desjenigen Behältnisses verstanden, dessen Innenoberfläche sterilisiert wird. Unter dem Mündungsbereich wird weiterhin der Bereich der Mündung und auch ggfs. der sich daran anschließende Kopfbereich des Behältnisses verstanden. Insbesondere wird auf diese Weise ein Gewindebereich des Behältnisses auch außen sterilisiert. Vorteilhaft werden daher dieje nigen Bereiche des Behältnisses sterilisiert, welche während des späteren Füllvorgangs mit der abzufüllenden Flüssigkeit oder dem Behältnisverschluss in Berührung kommen. Bevorzugt reichen der Kopf- bzw. Mündungsbereich der Kunststoffvorformlinge auch bis zu einem ggfs. an den Behältnissen angeordneten Tragring.

Bei den Behältnissen handelt es sich bevorzugt um Kunststoffbehältnisse, wobei unter dem Begriff Behältnis sowohl fertig gestellte Behältnisse wie z.B. Flaschen verstanden werden als auch etwa Kunststoffvorformlinge, welche insbesondere in Kunststoffbehältnisse umformbar sind.

Vorteilhaft wird der außenliegende Mündungsbereich durch die Bestrahlungseinrichtung sterilisiert. Besonders bevorzugt erfolgt diese Sterilisation des außenliegenden Mündungsbereichs unmittelbar vor oder unmittelbar nach der Sterilisierung der Innenoberflächen des Behältnisses. Vorteilhaft handelt es sich bei dem Behältnis um ein Kunststoffbehältnis.

Bei einem weiteren vorteilhaften Verfahren erfolgt die Sterilisation des Mündungsbereichs während die Bestrahlungseinrichtung vollständig außerhalb des Behältnisses angeordnet ist.

Bei einem besonders bevorzugten Verfahren weist die Bestrahlungseinrichtung einen stangenförmigen Körper auf, der in das Innere des Behältnisses - insbesondere durch die Mündung hindurch - eingeführt wird. In einem Endabschnitt dieses stangenförmigen Körpers kann dabei ein Austrittsfenster angeordnet sein, durch welches hindurch Elektronen aus dem stangenförmigen Körper austreten können.

Bei einem weiteren vorteilhaften Verfahren ist ein unteres Ende der Bestrahlungseinrichtung bzw. der besagte Endabschnitt während der Sterilisation des Mündungsbereiches in einem Abstand zu diesem angeordnet, der zwischen 0,5 cm und 10 cm, bevorzugt zwischen 1 cm und 6 cm und besonders bevorzugt zwischen 3 cm und 5 cm liegt. Durch diese genannten Abmessungen ist es möglich, dass die aus der Bestrahlungseinrichtung austretenden Ladungsträger, wie insbesondere Elektronen, noch über eine hinreichende Reichweite verfügen, um den Mündungsbereich und den sich daran anschließenden Kopfbereich des Behältnisses zu sterilisieren.

Bei einem weiteren vorteilhaften Verfahren werden unterhalb des Mündungs- und Kopfbereiches liegende Bereiche der Außenwandung des Behältnisses nicht durch Bestrahlung mit der Ladungsträgerstrahlung sterilisiert. In diesen Bereichen kann auf die Sterilisation verzichtet werden, da diese Bereiche nicht mit dem abzufüllenden Getränk in Berührung kommen können.

Bei einem weiteren vorteilhaften Verfahren werden die Behältnisse durch oder in einen Reinraum transportiert. So ist es möglich, dass sich ein derartiger Reinraum unmittelbar an die Bestrahlungseinrichtungen anschließt, es wäre jedoch auch möglich, dass die Bestrahlung bereits innerhalb eines Reinraums stattfindet und dieser Reinraum sich nach dem Sterilisationsvorgang fortsetzt. Bei einem weiteren vorteilhaften Verfahren bestrahlt die Bestrahlungseinrichtung den Mündungsbereich des Behältnisses zumindest solange, bis das Behältnis in einen Sterilraum eingetreten ist. Vorteilhaft kann dabei eine Grenze dieses Sterilraums jenseits (z. B. unterhalb) des oben beschriebenen Mündungsbereiches des Behältnisses, d. h. z. B. in Richtung des Behältnisbodens definiert werden. Dabei ist es möglich, dass das Behältnis eine Hubbewegung über die Sterilraumgrenze hinaus ausführt.

Bei einem weiteren vorteilhaften Verfahren wird das Behältnis mit einem gerichteten Strom eines gasförmigen Mediums beaufschlagt. So kann beispielsweise das Behältnis mit Sterilluft beaufschlagt werden, die insbesondere in einer Längsrichtung des Behältnisses strömt. Auf diese Weise kann die Sterilraumgrenze auch ohne physische Abtrennung unterhalb des besagten Mündungsbereiches des Behältnisses gehalten bzw. definiert werden. Vorteilhaft sind Luftfilter vorgesehen, durch welche hindurch die Sterilluft strömt.

Bei einem weiteren vorteilhaften Verfahren erfolgt die Bestrahlung wenigstens zeitweise, während sich die Bestrahlungseinrichtung von einem Boden des Behältnisses entfernt. Mit anderen Worten erfolgt die Bestrahlung vorteilhaft während eines Zurückziehens der Bestrahlungseinrichtung aus dem Behältnis. Dabei ist es möglich, dass die Bestrahlung erst einsetzt, sobald die Bestrahlungseinrichtung wieder aus dem Behältnis zurückgezogen wird. Es wäre jedoch auch möglich, dass die Bestrahlung sowohl während des Eintauchens als auch während des Herausziehens der Bestrahlungseinrichtung erfolgt.

Weiterhin ist es möglich, dass die Bestrahlung während des gesamten Herausziehvorgangs der Strahlungseinrichtung erfolgt, also insbesondere auch noch, wenn sich der Endabschnitt der Bestrahlungseinrichtung schon wieder außerhalb des Behältnisses befindet.

Das erfindungsgemäße Verfahren kann auf einer Vorrichtung zum Sterilisieren von Behältnissen ausgeführt werden, wobei die Vorrichtung eine Transporteinrichtung aufweist, welche die Behältnisse entlang eines vorgegebenen Transportpfads transportiert, sowie eine Bestrahlungseinrichtung, welche eine Ladungsträgerstrahlung und insbesondere eine Elektronenstrahlung abgibt, wobei diese Bestrahlungseinrichtung durch eine Relativbewegung zwischen dem Behältnis und der Bestrahlungseinrichtung in der Längsrichtung des Behältnisses in das Innere des Behältnisses einführbar ist, um eine Innenwandung des Behältnisses zu sterilisieren.

Erfindungsgemäß weist die Vorrichtung eine Steuerungseinrichtung zum Steuern der Bestrahlungseinrichtung auf, welche bewirkt, dass auch ein außenliegender Mündungsbereich des Behältnisses durch die von der Bestrahlungseinrichtung abgegebene Strahlung sterilisiert wird.

Bevorzugt ist in der Transportrichtung der Behältnisse nach der Vorrichtung zum Sterilisieren der Behältnisse eine weitere Vorrichtung zum Behandeln von Behältnissen angeordnet. Bei dieser weiteren Vorrichtung kann es sich beispielsweise um eine Fülleinrichtung handeln, welche die Behältnisse insbesondere mit einer Flüssigkeit, wie beispielsweise einem Getränk befüllt. Daneben kann es sich jedoch bei der weiteren Maschine auch um eine Maschine handeln, welche Kunststoffvorformlinge behandelt, etwa eine Umformungseinrichtung, welche die Kunststoffvorformlinge zu Kunststoffbehältnissen umformt oder auch eine Erwärmungseinrichtung, welche Kunststoffvorformlinge erwärmt.

Es wird daher auch vorrichtungsseitig vorgeschlagen, dass die genannte Bestrahlung auch aktiviert wird, während sich die Bestrahlungseinrichtung zumindest teilweise und bevorzugt vollständig außerhalb des Behältnisses befindet.

Vorteilhaft weist die Bestrahlungseinrichtung an einer Stirnseite ein Austrittsfenster auf, durch welches hindurch die Ladungsträger bzw. Elektronen aus der Bestrahlungseinrichtung austreten können.

Vorteilhaft bewegt sich diese Strahlungseinrichtung wenigstens abschnittsweise gemeinsam mit den Behältnissen entlang des Transportpfades derselben mit. So ist es möglich, dass beispielsweise eine Trägereinrichtung vorgesehen ist, an der eine Vielzahl von Bestrahlungseinrichtungen angeordnet ist, sowie auch eine Vielzahl von Transporteinrichtungen zum Transportieren der Behältnisse. Während dieses Transports der Behältnisse können damit die Bestrahlungseinrichtungen, die hier beispielsweise stangenartige Körper aufweisen, in das Innere der Behältnisse eingeführt werden, um diese zu sterilisieren.

Bei einer weiteren vorteilhaften Ausführungsform weist die Vorrichtung eine und bevorzugt eine Vielzahl von Beschleunigungseinrichtungen auf, welche die Elektronen in Richtung des besagten Austrittsfensters der Bestrahlungseinrichtung beschleunigen. Bei einer weiteren vorteilhaften Ausführungsform weist die Vorrichtung auch eine Beaufschlagungseinrichtung auf, um die Behältnisse mit einer Strömung eines gasförmigen Mediums zu beaufschlagen.

Vorteilhaft werden die Behältnisse während ihrer Sterilisation entlang wenigstens eines abschnittsweise kreisförmigen Transportpfades geführt.

Bei einer weiteren vorteilhaften Ausführungsform weist die Vorrichtung einen Sterilraum auf, durch den die Kunststoffbehältnisse geführt werden. Dabei ist es möglich, dass dieser Sterilraum kanalförmig um den Transportpfad der Behältnisse ausgebildet ist. Insbesondere wäre es dabei denkbar, dass der besagte Sterilraum ringförmig um den Transportpfad der Behältnisse ausgeführt ist. Auf diese Weise ist es möglich, das Volumen des besagten Sterilraums relativ klein zu halten, um auf diese Weise eine kostengünstige Aufrechterhaltung der sterilen Bedingungen zu ermöglichen.

Weitere Vorteile und Ausführungsformen ergeben sich aus den beigefügten Zeichnungen. Darin zeigen:
- Fig. 1: Eine schematische Darstellung eines Sterilisationsvorgangs;
- Fig. 2: eine weitere schematische Darstellung eines Sterilisationsvorgangs;
- Fig. 3: eine weitere schematische Darstellung eines Sterilisationsvorgangs;
- Fig. 4: eine Darstellung des Sterilisationsvorgangs mit zusätzlicher Luftbeaufschlagung;
- Fig. 5: eine schematische Darstellung eines Sterilisationsvorgangs mit Transport der Behältnisse;
- Fig. 6: eine weitere mögliche Ausführungsform einer Sterilisationseinrichtung für Behältnisse;
- Fig. 7: eine schematische Darstellung eines Sterilisationsverlaufs; und
- Fig. 8a-8d: vier Detaildarstellungen des in Fig. 7 gezeigten Sterilisationsverlaufs.

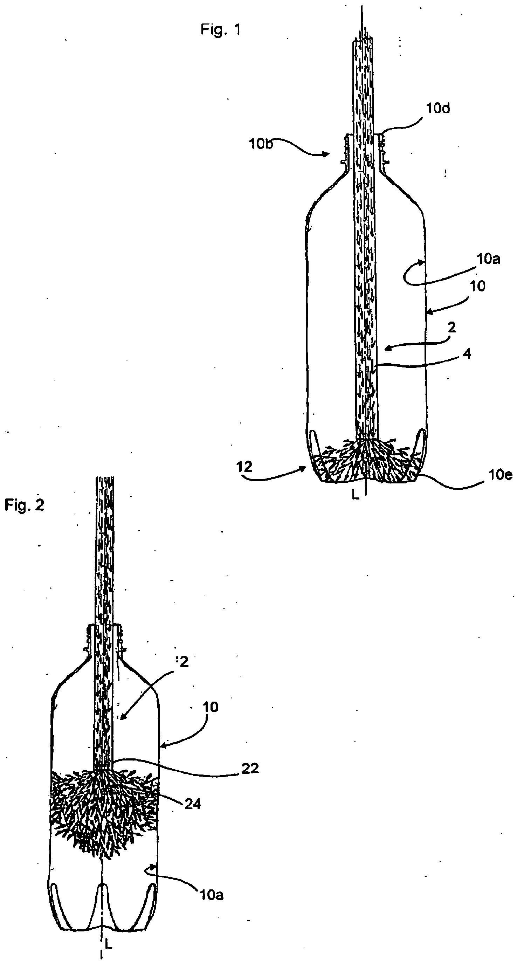

Die Figuren 1-3 zeigen schematisch drei Darstellungen zur Veranschaulichung des Sterilisationsprozesses. Dabei ist eine (nur schematisch dargestellte) Sterilisationseinrichtung 2 vorgesehen, die hier als stangenförmiger Körper ausgebildet ist, der durch eine Mündung 10d in das Innere des Behältnisses 10 einführbar ist. Bei der in Fig. 1 gezeigten Situation ist die Bestrahlungseinrichtung fast bis zum Boden 10e des Behältnisses in dieses eingefahren und kann auf diese Weise auch den besagten Bodenbereich 10e mit einer Elektronenwolke 12 beaufschlagen. Durch diese Beaufschlagung wird die in ihrer Gesamtheit mit 10a gekennzeichnete Innenwandung des Kunststoffbehältnisses sterilisiert. Die Elektronen 4 werden dabei durch eine (nicht gezeigte) Beschleunigungseinrichtung beschleunigt. Das Bezugszeichen 10b kennzeichnet einen die Mündung 10d enthaltenden Mündungsbereich, der sich jedoch in der Längsrichtung L des Behältnisses weiter (nach unten) erstreckt als die Mündung 10d.

Fig. 2 zeigt eine zweite Situation, bei der der untere Endabschnitt 22 mit dem Austrittsfenster 24 in einem mittleren Bereich des Behältnisses 10 angeordnet ist. In diesem Fall wird ein mittlerer Bereich der Innenwandung sterilisiert.

Es ist dabei möglich, dass die Bewegung der Bestrahlungseinrichtung 2 gegenüber dem Behältnis 10 mit konstanter Geschwindigkeit erfolgt, es wäre jedoch auch möglich, dass die Geschwindigkeit beispielsweise von einer Kontur des Behältnisses abhängt und bei Bereichen mit größeren Querschnitten beispielsweise eine langsamere Relativbewegung erfolgt sowie bei Bereichen mit einem geringeren Querschnitt eine schnellere Bewegung, um auf dieses Weise eine jeweils konstante Strahlungsdosis auf die Innenwandung 10a des Behältnisses aufzubringen.

Bei allen Ausgestaltungen erfolgt dabei die Relativbewegung zwischen den Behältnissen 10 und der Bestrahlungseinrichtung 2 in einer Längsrichtung L des Behältnisses 10.

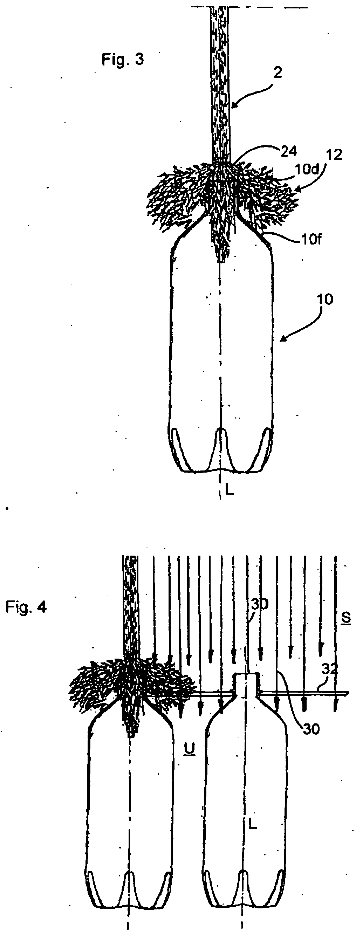

Fig. 3 veranschaulicht eine weitere Position der Bestrahlungseinrichtung 2 gegenüber dem Behältnis 10. Hier ist die Bestrahlungseinrichtung 2 bzw. auch das Austrittsfenster 24 vollständig aus dem Behältnis 10 herausgezogen. Die Elektronenwolke 12 beaufschlagt hier die Mündung 10d des Behältnisses sowohl von innen als auch von außen, und bewirkt damit eine Sterilisation dieser Mündung. Daneben wird jedoch auch der Mündungsbereich mit Elektronen beaufschlagt und hier auch noch ein oberer Behältnisbereich 10f.

Fig. 4 veranschaulicht die in Fig. 3 gezeigte Situation, wobei hier zusätzlich noch eine gerichtete Luftströmung 30 auf die Behältnisse gerichtet wird. Genauer erfolgt die Beaufschlagung mit der Sterilluft hier ebenfalls in der Längsrichtung L des Behältnisses, um auf diese Weise ein Wandern der Sterilraumgrenze zu vermeiden. Das Bezugszeichen U kennzeichnet den unsterilen Bereich und das Bezugszeichen S den sterilen Bereich. Das Bezugszeichen 32 kennzeichnet die Sterilraumgrenze, wobei diese hier nicht notwendigerweise durch eine Materialgrenze ausgebildet sein muss. Es wäre jedoch auch möglich, dass die Luftströmung hier auch schräg bezüglich der Längsrichtung der Kunststoffbehältnisse auf diese auftrifft. Bevorzugt weist jedoch diese Luftströmung 30 auch eine Komponente auf, die in der Längsrichtung L - insbesondere von der Mündung der Behältnisse zum Boden der Behältnisse - verläuft.

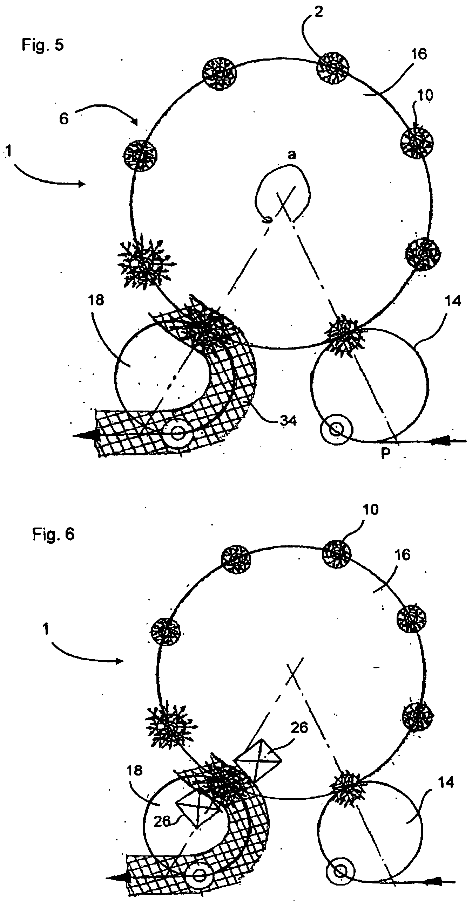

Fig. 5 zeigt eine grob schematische Veranschaulichung einer erfindungsgemäßen Vorrichtung 1. Die Vorrichtung 1 weist hier ein Trägerrad 16 auf, an dem eine Vielzahl von Transportelementen (nicht gezeigt) zum Halten der Behältnisse 10 angeordnet ist. Dieses Trägerrad ist damit Teil einer Transporteinrichtung 6 zum Transportieren von Behältnissen. Weiterhin ist an diesem Trägerrad 16 eine Vielzahl von Bestrahlungseinrichtungen 2 angeordnet, die wie in der vorher gezeigten Weise in die Mündungen der Kunststoffbehältnisse eingeführt werden können. Das Bezugszeichen P kennzeichnet den Transportpfad der Behältnisse und das Bezugszeichen a den Behandlungswinkel für die Sterilisation. Zunächst werden unsterile Behältnisse 10 über ein Einlaufrad 14 an das Trägerrad 16 gegeben. Während des Transports mit dem Trägerrad 16 werden die Kunststoffbehältnisse zunächst an ihrer Innenwandung sterilisiert. Beim Auslaufen der Behältnisse bzw. der Übergabe an das Auslaufrad 18 werden diese auch in ihrem Mündungsbereich und damit auch außen sterilisiert.

Das Bezugszeichen 34 kennzeichnet einen Sterilraum, in dem die Behältnisse einlaufen, bereits während sie noch sterilisiert werden. Anschließend werden die nunmehr sterilen Behältnisse durch den hier kanalförmigen Sterilraum 34 ausgeführt. In diesem Bereich des Sterilraums ist es wie oben erwähnt auch möglich, dass lediglich oberhalb eines bestimmten Bereichs der Behältnisse, beispielsweise oberhalb eines Halsrings eine sterile Zone ausgebildet ist, unterhalb derselben jedoch nicht.

Fig. 6 zeigt eine weitere Ausführungsform einer erfindungsgemäßen Vorrichtung. Im Gegensatz zu der in Fig. 5 gezeigten Ausführungsform ist hier eine zusätzliche Sterilisationseinrichtung 26 vorgesehen, welche den Mündungsbereich der Kunststoffbehältnisse, hier insbesondere ebenfalls durch Beaufschlagung mit Elektronen, sterilisiert. Daneben wären hier jedoch auch andere Vorgehensweisen der Sterilisation denkbar, etwa eine Sterilisation mit UV-Strahlung oder mit Röntgenstrahlung, mit Gammastrahlung und dergleichen. Diese Sterilisationseinrichtung 26 ist hier stationär angeordnet.

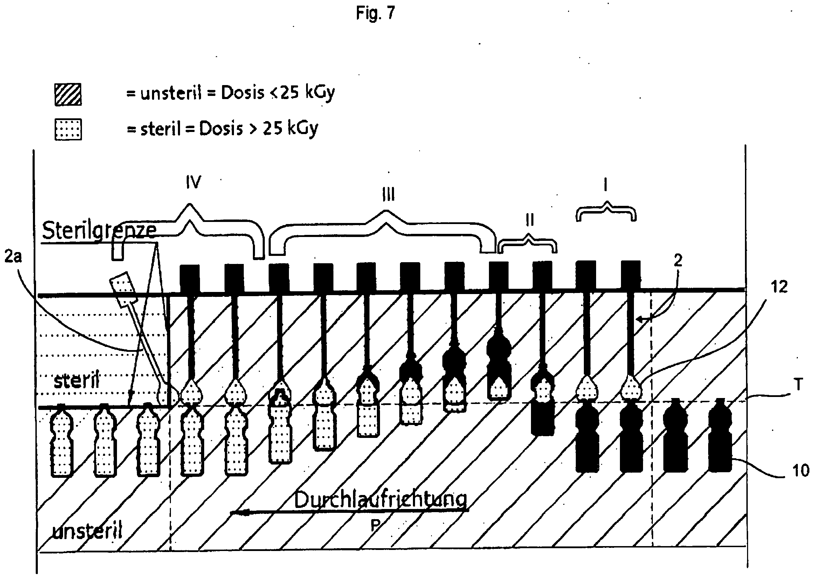

Fig. 7 zeigt eine weitere Darstellung zur Veranschaulichung des Sterilisationsvorgangs. Hier ist eine Vielzahl von Bestrahlungseinrichtungen 2 vorgesehen, die hier gemeinsam mit den Behältnissen 10 entlang des Transportpfades P transportiert werden. Dieser Transportpfad P kann dabei, wie oben erwähnt, sowohl kreisförmig, als auch geradlinig sein oder andere Bewegungsrichtungen aufweisen. Man erkannt hier, dass die einzelnen Bestrahlungseinrichtungen fest ausgebildet sind, d. h. sich nicht in der Längsrichtung der Behältnisse bewegen. Vielmehr werden die Behältnisse hier angehoben, sodass die Bestrahlungseinrichtungen 2 in das Innere der Behältnisse eindringen, so dass hier eine Sterilisation stattfinden kann.

Die gestrichelte Linie T kennzeichnet hier eine Trennlinie zwischen einem sterilen Bereich und einem unsterilen Bereich. In dem sterilen Bereich kann eine Strahlungsdosis aufgebracht werden, welche oberhalb 25 kGy liegt und in dem unsterilen Bereich liegt die Dosis entsprechend unter 25 kGy. Der Sterilisationsprozess kann hier in mehrere Abschnitte I-IV unterteilt werden. In dem Abschnitt I werden die Behältnisse nicht entlang ihrer Längsrichtung angehoben oder gesenkt, und in diesem Bereich werden die Mündungen der Behältnisse mit der Elektronenwolke 12 beaufschlagt, sodass hier eine Sterilisation stattfindet. In dem Bereich II werden die Behältnisse nach oben angehoben, sodass die Bestrahlungseinrichtung 2 in das Innere der Behältnisse eindringt. Auf diese Weise ist eine Sterilisation des Bodenbereiches und auch eines mittleren Bereiches des Behältnisses möglich.

In dem Abschnitt III werden die Behältnisse anschließend wieder abgesenkt, wobei auch in diesem Abschnitt, wie in der Fig. 7 gezeigt, eine Sterilisation der Innenoberfläche der Behältnisse stattfindet. In den beiden rechten Zuständen von Abschnitt III findet jedoch bei dem bereits fast wieder abgesenkten Behältnis auch eine Bestrahlung des äußeren Mündungsbereiches des Behältnisses statt. Man erkennt, dass die Behältnisse schneller angehoben als anschließend wieder abgesenkt werden. Bevorzugt wird daher die Bestrahlungseinrichtung 2 schneller in die Behältnisse eingeführt als sie anschließend wieder aus diesen entfernt wird.

In dem Abschnitt IV werden die Behältnisse wieder in ihrer unteren Position geführt und dabei in ihrem Mündungsbereich bestrahlt. Dabei kann auch eine Bestrahlungseinrichtung 2a vorgesehen sein, welche schräg ausgerichtet oder allgemein in anderer Weise angeordnet ist und die Behältnisse von der Seite her bestrahlt. Weiterhin wäre es auch möglich, dass die Kunststoffbehältnisse während der Innenbestrahlung auch um ihre Längsachse gedreht werden um so eine noch gleichmäßigere Verteilung der Elektronen auf der Innenoberfläche der Behältnisse zu erreichen.

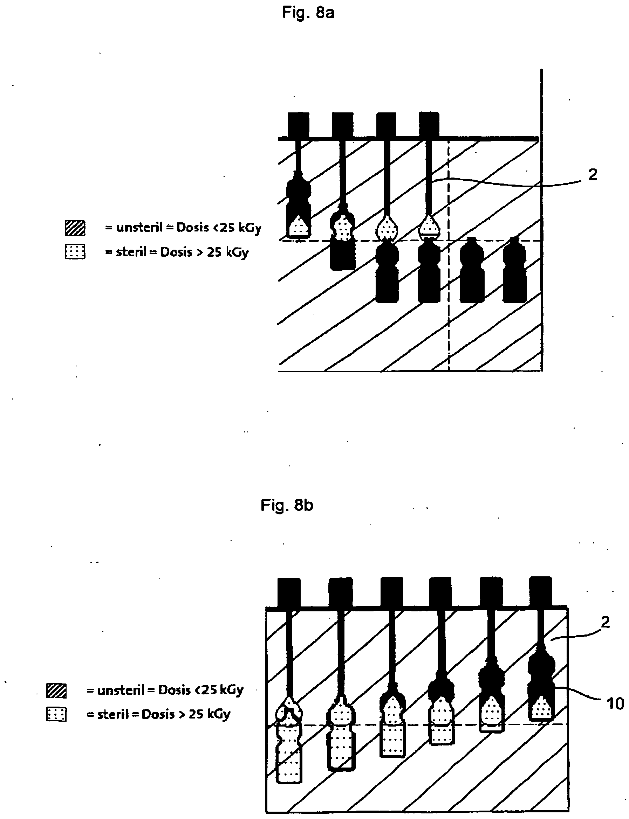

Die Figuren 8a bis 8b zeigen die einzelnen Abschnitte in detailierterer Darstellung. Dabei wäre es bei der in Fig. 8a gezeigten Situation möglich, dass die Behältnisse beispielsweise über ein Schleusenrad einlaufen, wobei bevorzugt die bestehende Gammastrahlung abgeschirmt wird. Weiterhin werden hier die Behältnisse über die Elektronenstrahleinrichtung 2 angehoben, wobei bevorzugt zunächst noch keine Entkeimung der inneren Oberfläche stattfindet. Diese beginnt in Fig. 8b erst bei den beiden links dargestellten Situationen.

Bei der in Fig. 8b gezeigten Situation wird das Behältnis gegenüber der Bestrahlungseinrichtung 2 sukzessive abgesenkt und in diesem Zeitraum auch eine minimale Dosis auf die Innenoberfläche des Behältnisses aufgebracht. Kritisch ist hier das Auftreten von Arcs (Aussetzern der Elektronenbestrahlung). Bei Auftreten von Arcs ist die Dosis auf der Oberfläche der Behälterinnenseite zu gering. Falls insbesondere mittels Sensoreinrichtungen ein derartiges Auftreten von Arc festgestellt wird, ist es möglich, dass die Behältnisse nicht in den nachfolgenden Sterilbereich übergeben, sondern erneut behandelt oder ausgeschleust werden. Die benötigte Behandlungsdauer für die Elektronenstrahlendosis kann über die Absenk- oder Hubgeschwindigkeit der Behältnisse reguliert werden. Die Grenzen einer zulässigen Dosis ergeben die Notwendigkeit eines angepassten Verfahrprofils für das Senken der Behältnisse und die notwendige Eintauchtiefe.

Für das Anheben und Senken der Behältnisse können beispielsweise Antriebe wie mechanische Hubkurven Einsatz finden. Daneben wäre es auch möglich, dass Linearmotoren, Elektromotoren, hydraulische Antriebe oder pneumatische Antriebe Verwendung finden, um die Behältnisse anzuheben. Es wäre jedoch umgekehrt auch möglich, nicht die Behältnisse selbst zu heben und zu senken, sondern anstelle dessen (oder zusätzlich) die einzelnen Bestrahlungseinrichtungen 2.

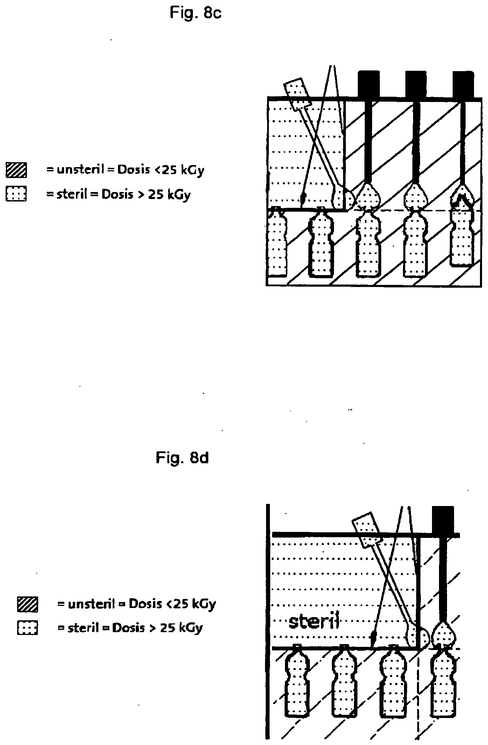

Wie Fig. 8b zu entnehmen ist, findet keine Entkeimung der Behältnisaußenseite statt, sodass per Definition der Bereich außerhalb des Behältnisses unsteril ist. Bei der in Fig. 8c gezeigten Situation befindet sich das Behältnis unter der Bestrahlungseinrichtung 2. Hier wird zumindest eine minimale Dosis auf die Außenseite des Behältnisses oder Gebindes aufgebracht.

Bei dem Eintauchen der Bestrahlungseinrichtung in die Behältnisse muss noch nicht zwingend eine Sterilisationswirkung auftreten. Dabei kann hier die Bestrahlung ein oder auch ausgeschaltet sein. Nach Definition der Sterilisation können sich oberhalb des Austrittsfensters, an dem die Strahlenwolke 12 steht wieder Rekontaminationen ergeben, beispielsweise dadurch, dass wie oben durch die Mündung Keime eingeschleppt werden. Gleichwohl bringt jedoch eine Bestrahlung von oben nach unten eine zusätzliche Sicherheit, wie in Fig. 8a veranschaulicht.

Die eigentliche Sterilisation beginnt, wie in Fig. 8b dargestellt, vom Boden des Behältnisses im Inneren des Behältnisses aus durch Relativbewegungen der Strahlungseinrichtung in Richtung der Mündung des Behältnisses. Dabei kann, wie oben erwähnt, diese Relativbewegung der Behälterwand, beispielsweise einer Wanddicke oder auch einem Wanddurchmesser angepasst sein. Vorteilhaft hat dabei jeglicher Gasaustausch innerhalb des Behältnisses, der während der Entkeimung in dem Behältnis zwischen dem sterilen Bereich und dem unsterilen Bereich entsteht zur Folge, dass alle diese Bereiche durch die Strahlenwolke 12 wandern müssen und somit sterilisiert werden, wie in Fig. 8b veranschaulicht.

In dem dritten Schritt wird die Bestrahlungseinrichtung aus dem Behältnis gezogen und über der Mündung derart positioniert oder bewegt, dass die Mündung im Außenbereich bevorzugt bis zum Tragring sterilisiert wird und insbesondere gleichzeitig die Öffnung der Mündung derart bestrahlt wird, dass alle Partikel, die in die Behältnisse fallen wiederum durch die sterilisierende Wolke 12 treten müssen. Durch diesen Schutz in der Strahlenwolke kann die Sterilisation in einer unsterilen Umgebung geschehen und auch aufrecht erhalten werden.

Fig. 8c zeigt diese Aufrechterhaltung des Sterilisationszustandes. Es wird auch hier der Austritt von Mikroorganismen bei einem vorherigen Arc während der Entkeimung der Flascheninnenseite verhindert. Wie oben indiziert auch hier das Auftreten von Arc eine zu niedrige Dosis auf dem Neckring außen. Das Behältnis wird nicht in den Sterilbereich übergeben, sondern erneut behandelt bzw. ausgeschleust.

Weiterhin wird bevorzugt die Behandlungsdauer für die Minimaldosis oder die Dauer der Verweilzeit in dem Elektronenstrahl so geregelt, dass eine Überdosierung anderer Oberflächen vermieden wird.

Fig. 8d zeigt einen weiteren Schritt der Behältnisentkeimung. Hier ist das Behältnis innen und auch im Mündungsbereich sterilisiert und wird durch die gleichwohl noch aufgebrachte Strahlenwolke 12 vor einer Rekontamination geschützt. Anschließend wird das Behältnis in einen Sterilraum übergeben, der sich nur im Bereich der Mündung des Behältnisses erstreckt. Derartige Neckringisolatoren sind im Stand der Technik bekannt, wobei bei diesen Sterilisatoren lediglich ein kleiner Bereich, der nur um und über der Mündung liegt, steril ist. Wie oben erwähnt, kann dabei eine gerichtete Sterilluftbewegung, bevorzugt von oben nach unten, Einsatz finden, um die Steriliität sicher zu stellen.

Zur Übergabe aus der Strahlenwolke in die Sterilluftströmung können zusätzliche Maßnahmen getroffen werden, wie beispielsweise Strahlenemitter, Luftdüsen, UV-Lampen oder Plasma.

Nach dem Sterilisationsvorgang können die Behältnisse in ein Schleusenrad oder einen Transferstern im Auslauf übergeben werden. Dabei werden bevorzugt die Arcs der rotierenden Emitter während der Innen- und Neckbehandlung durch statische Emitter abgesichert. Die Absicherung gegenüber Kontamination der sterilen Zone wird ebenfalls bevorzugt - insbesondere durch eine Bestrahlungseinrichtung - durchgeführt.

Eine Minimaldosis auf der Außenseite des Behältnisses kann, wie oben erwähnt, auch durch statische Emitter erreicht werden.

Hinsichtlich der Arcs sind jedoch statische Emitter teilweise kritisch, insbesondere wenn während der Behandlung innen oder im Mündungsbereich bereits Arc an einem rotierendem Emitter aufgetreten ist.

Im Auslauf können die Behältnisse wiederum über ein Schleusenrad abgeführt werden, wobei auch hier wiederum eine Abschirmung der Gammastrahlung erfolgt.

Die Transporteinrichtung zum Transportieren der Behältnisse kann die Behältnisse beispielsweise an deren Boden führen, es wäre jedoch auch möglich, dass die Transporteinrichtung die Behältnisse an deren Mündungen bzw. an deren Tragring führt. Auch wäre ein Transport über die Seitenwandungen der Behältnisse denkbar.

Im Rahmen einer Nachbehandlung können die Behältnisse mit steriler Luft gespült werden. Dabei kann beispielsweise Ozon ausgeblasen werden, welches bei der Behandlung entstanden ist.

Die Anmelderin behält sich vor, sämtliche in den Anmeldungsunterlagen offenbarten Merkmale als erfindungswesentlich zu beanspruchen, sofern sie einzeln oder in Kombination gegenüber dem Stand der Technik neu sind.

### Bezugszeichenliste

- 1: Vorrichtung
- 2: Bestrahlungseinrichtung, Elektronenstrahleinrichtung
- 2a: Bestrahlungseinrichtung
- 4: Elektronen
- 6: Transporteinrichtung
- 10: Behältnis
- 10a: Innenwand
- 10b: Mündungsbereich
- 10d: Mündung
- 10e: Boden
- 10f: Behältnisbereich
- 12: Elektronenwolke, Strahlenwolke
- 14: Einlaufrad
- 16: Trägerrad
- 18: Auslaufrad
- 22: Endabschnitt
- 24: Austrittsfenster
- 26: Sterilisationseinrichtung
- 30: Luftströmung
- 32: Sterilraumgrenze
- 34: Sterilraum
- L: Längsrichtung
- P: Transportpfad
- U: unsteriler Bereich
- S: steriler Bereich
- I-IV: Abschnitte
- a: Behandlungswinkel
- T: Trennlinie

## Patentansprüche

1. Verfahren zum Sterilisieren von Behältnissen (10) mittels Elektronenstrahlung wobei die Behältnisse (10) von einer Transporteinrichtung (6) entlang eines vorgegebenen Transportpfades (P) transportiert werden und wobei eine Bestrahlungseinrichtung (2) durch eine Relativbewegung zwischen dem Behältnis (10) und der Bestrahlungseinrichtung (2) in einer Längsrichtung (L) des Behältnisses (10) in das Innere des zu sterilisierenden Behältnisses (10) eingeführt wird und eine Ladungsträgerstrahlung (12) umfassend Elektronenstrahlung abgibt, um mittels dieser Ladungsträgerstrahlung (12) die Innenwandung (10a) des Behältnisses (10) zu sterilisieren,
**dadurch gekennzeichnet, dass**
auch ein außenliegender Gewindebereich (10b) des Behältnisses (10) durch die Bestrahlungseinrichtung (2) sterilisiert wird, wobei die Steritisation des außenliegenden Gewindebereichs (10b) unmittelbar vor oder unmittelbar nach der Sterilisierung der Innenoberflächen mittels dieser Bestrahlungseinrichtung (2) des Behältnisses (10) erfolgt.

2. Verfahren nach Anspruch 1,
**dadurch gekennzeichnet, dass**
die Sterilisation des Mündungsbereichs (10b) erfolgt, während die Bestrahlungseinrichtung (2) vollständig außerhalb des Behältnisses (10) angeordnet ist.

3. Verfahren nach Anspruch 2,
**dadurch gekennzeichnet, dass**
ein unteres Ende der Bestrahlungseinrichtung (2) während der Sterilisation des Mündungsbereichs (10b) in einem Abstand angeordnet ist, der zwischen 0,5cm und 10cm, bevorzugt zwischen 1 cm und 6cm und besonders bevorzugt zwischen 3cm und 5cm liegt.

4. Verfahren nach wenigstens einem der vorangegangenen Ansprüche,
**dadurch gekennzeichnet, dass**
unterhalb des Mündungs- und Kopfbereichs liegende Bereiche der Außenwandung des Behältnisses (10) nicht durch Bestrahlung mit der Ladungsträgerstrahlung sterilisiert werden.

5. Verfahren nach wenigstens einem der vorangegangenen Ansprüche,
**dadurch gekennzeichnet, dass**
die Behältnisse (10) durch oder in einen Reinraum transportiert werden.

6. Verfahren nach wenigstens einem der vorangegangenen Ansprüche,
**dadurch gekennzeichnet, dass**
die Bestrahlungseinrichtung (2) den Mündungsbereich (10b) des Behältnisses zumindest so lange bestrahlt, bis das Behältnis (10) in einen Sterilraum eingetreten ist.

7. Verfahren nach wenigstens einem der vorangegangenen Ansprüche,
**dadurch gekennzeichnet, dass**
das Behältnis (10) mit einem gerichteten Strom eines gasförmigen Mediums beaufschlagt wird.

8. Verfahren nach wenigstens einem der vorangegangenen Ansprüche,
**dadurch gekennzeichnet, dass**
die Bestrahlung wenigstens zeitweise erfolgt, während sich die Bestrahlungseinrichtung (2) von einem Boden (10e) des Behältnisses (10) entfernt.

## Claims

1. A method for sterilizing containers (10) by means of electron beam radiation, wherein the containers (10) are transported along a pre-set transport path (P) by a transport device (6) and wherein an irradiation device (2) is introduced by a relative movement between the container (10) and the irradiation device (2) in a longitudinal direction (L) of the container (10) into the interior of the container (10) to be sterilized and emits a charge carrier irradiation (12) comprising electron beam radiation in order to sterilize the inner wall (10a) of the container (10) by means of this charge carrier irradiation (12),
**characterized in that**
a thread region (10b) - situated on the outside - of the container (10) is also sterilized by the irradiation device (2), wherein the sterilization of the thread region (10b) situated on the outside is carried out directly before or directly after the sterilization of the inner surfaces by means of this irradiation device (2) of the container (10).

2. A method according to claim 1,
**characterized in that**
the sterilization of the aperture region (10b) is carried out while the irradiation device (2) is situated completely outside the container (10).

3. A method according to claim 2,
**characterized in that**
during the sterilization of the aperture region (10b) a lower end of the irradiation device (2) is situated at a distance which is between 0.5cm and 10cm, preferably between 1 cm and 6cm and in a particularly preferred manner between 3cm and 5cm.

4. A method according to at least one of the preceding claims,
**characterized in that**
regions of the outer wall of the container (10) which are situated below the aperture and head region are not sterilized by irradiation with the charge carrier irradiation.

5. A method according to at least one of the preceding claims,
**characterized in that**
the containers (10) are transported through or into a clean room.

6. A method according to at least one of the preceding claims,
**characterized in that**
the irradiation device (2) irradiates the aperture region (10b) of the container at least until the container (10) has entered a sterile room.

7. A method according to at least one of the preceding claims,
**characterized in that**
the container (10) is acted upon with a directed flow of a gaseous medium.

8. A method according to at least one of the preceding claims,
**characterized in that**
the irradiation takes place at least for a time, while the irradiation device (2) is removed from a base (10e) of the container (10).

## Revendications

1. Procédé destiné à la stérilisation de récipients (10) au moyen d'un rayonnement électronique, dans lequel les récipients (10) sont transportés par un dispositif de transport (6) le long d'un chemin de transport (P) prédéfini et dans lequel un dispositif d'exposition à un rayonnement (2) est introduit à l'intérieur du récipient (10) à stériliser par un déplacement relatif entre le récipient (10) et le dispositif d'exposition à un rayonnement (2) dans un sens longitudinal (L) du récipient (10) et émet un rayonnement porteur de charge (12) comprenant un rayonnement électronique afin de stériliser la paroi intérieure (10a) du récipient (10) au moyen dudit rayonnement porteur de charge (12),
**caractérisé en ce qu'**
une zone de filetage (10b), située à l'extérieur, du récipient (10) est stérilisée par le dispositif d'exposition à un rayonnement (2), dans lequel la stérilisation de la zone de filetage (10b) située à l'extérieur est effectuée directement avant ou directement après la stérilisation des surfaces intérieures au moyen dudit dispositif d'exposition à un rayonnement (2) du récipient (10).

2. Procédé selon la revendication 1,
**caractérisé en ce que**
la stérilisation de la zone d'embouchure (10b) est effectuée, tandis que le dispositif d'exposition à un rayonnement (2) est disposé totalement à l'extérieur du récipient (10).

3. Procédé selon la revendication 2,
**caractérisé en ce qu'**
une extrémité inférieure du dispositif d'exposition à un rayonnement (2) est disposée pendant la stérilisation de la zone d'embouchure (10b) à une distance, qui est comprise entre 0,5cm et 10cm, de manière préférée entre 1 cm et 6cm, et de manière particulièrement préférée entre 3cm et 5cm.

4. Procédé selon au moins l'une quelconque des revendications précédentes,
**caractérisé en ce que**
des zones, situées sous la zone d'embouchure et de tête, de la paroi extérieure du récipient (10) ne sont pas stérilisées par l'exposition au rayonnement porteur de charge.

5. Procédé selon au moins l'une quelconque des revendications précédentes,
**caractérisé en ce que**
les récipients (10) sont transportés à travers ou dans une salle blanche.

6. Procédé selon au moins l'une quelconque des revendications précédentes,
**caractérisé en ce que**
le dispositif d'exposition à un rayonnement (2) expose à un rayonnement la zone d'embouchure (10b) du récipient au moins jusqu'à ce que le récipient (10) soit entré dans un espace stérile.

7. Procédé selon au moins l'une quelconque des revendications précédentes,
**caractérisé en ce que**
le récipient (10) est soumis à l'action d'un flux orienté d'un milieu sous forme gazeuse.

8. Procédé selon au moins l'une quelconque des revendications précédentes,
**caractérisé en ce que**
l'exposition à un rayonnement est effectuée au moins par intermittence, alors que le dispositif d'exposition à un rayonnement (2) s'éloigne d'un fond (10e) du récipient (10).
